# EUROPÄISCHE PATENTANMELDUNG

(11) **EP 3 195 899 A1**
(43) Veröffentlichungstag der Anmeldung: **26.07.2017**
(21) Anmeldenummer: 17151701.4
(22) Anmeldetag: 17.01.2017
(51) Int. Cl.: A61N 1/375

(54) **DURCHFÜHRUNG EINES MEDIZINELEKTRONISCHEN GERÄTS, VERFAHREN ZUR HERSTELLUNG EINER SOLCHEN UND MEDIZINELEKTRONISCHES GERÄT**

(30) Priorität: 20.01.2016 DE 102016100865
(71) Anmelder: BIOTRONIK SE & Co. KG, 12359 Berlin (DE)
(72) Erfinder: Sontheimer, Thomas, 90574 Roßtal (DE); Zecho, Kathrin, 91369 Wiesenthau (DE); Teske, Josef, 96103 Hallstadt (DE)
(74) Vertreter: Keck, Hans-Georg

(57) **Zusammenfassung**

Durchführung eines, insbesondere implantierbaren, medizinelektronischen Gerätes mit einem Gehäuse und mindestens einem in dem Gehäuse aufgenommenen elektrischen oder elektronischen Bauelement, wobei die Durchführung einen Durchführungs-Flansch zum Verschluss einer Öffnung des Gehäuses und zur Aufnahme einer Mehrzahl von Anschlusselementen, die zum gehäuse-externen Anschluss des oder mindestens eines Bauelementes dienen, in einem die Anschlusselemente umgebenden Isolierkörper aufweist, wobei der Isolierkörper aus Mehrlagenkeramik insbesondere aus HTCC, mit eingesinterten vorkonfigurierten Leiterelementen ausgebildet und mit einer gerätespezifischen Kontaktierung ausgewählter Leiterelemente versehen ist, die zusammen mit den kontaktierten vorkonfigurierten Leiterelementen die Anschlusselemente bildet.

## Beschreibung

Die Erfindung betrifft eine Durchführung eines implantierbaren medizinelektronischen Gerätes sowie auch ein derartiges Gerät. Dieses umfasst typischerweise ein Gerätegehäuse, in dem elektronische und elektrische Funktionseinheiten untergebracht sind, und die Durchführung weist einen Durchführungs-Flansch zum Verschluss einer Öffnung des Gehäuses und zur Aufnahme einer Mehrzahl von Anschlusselementen, die zum gehäuse-externen Anschluss des oder mindestens eines Bauelementes dienen, in einem die Anschlusselemente umgebenden Isolierkörper auf Des Weiteren betrifft die Erfindung ein Verfahren zur Herstellung einer solchen Durchführung.

Implantierbare Geräte der oben bezeichneten Art sind insbesondere als Herzschrittmacher oder implantierbare Kardioverter (speziell Defibrillatoren) seit langem in massenhaftem Einsatz. Es kann sich hierbei aber auch um eine weniger komplexe Vorrichtung, wie etwa eine Elektroden- oder Sensorleitung oder auch ein Cochlearimplantat, handeln.

Die meisten praktisch bedeutsamen implantierbaren medizinelektronischen Geräte sind dazu vorgesehen, über geeignet platzierte Elektroden elektrische Impulse an reizbares Körpergewebe abzugeben. Um diese Funktion auszuführen, sind im Gehäuse des Gerätes elektronische/elektrische Funktionseinheiten zur Erzeugung der Impulse und zur geeigneten Steuerung der Impulserzeugung untergebracht, und außen am Gerät sind unmittelbar Elektroden oder Anschlüsse für mindestens eine Elektrodenleitung vorgesehen, in deren distalem Endabschnitt die Elektroden zur Impulsübertragung an das Gewebe angebracht sind.

Die elektronischen/elektrischen Funktionseinheiten im Geräteinneren sind in einer Weise mit den äußeren Elektroden oder Elektrodenleitungsanschlüssen zu verbinden, die unter den speziellen Bedingungen des implantierten Zustandes eine absolut und dauerhaft verlässliche Funktion gewährleistet. Des Weiteren hat die Durchführung eines solchen Gerätes dessen dauerhafte Dichtigkeit unter diesen Bedingungen zu sichern.

Bekannt sind insbesondere Durchführungen, deren Grund- und Isolationskörper im Wesentlichen aus Keramik oder Glas besteht, wobei auch mehrschichtige bzw. mehrteilige Aufbauten unter Einsatz von Metallen oder Metalloxiden entwickelt wurden und eingesetzt werden. Derartige bekannte Durchführungen erfüllen weitgehend die an sie gestellten Anforderungen.

Ein Durchführungs-Flansch kann durch abtragende Materialbearbeitung (Fräsen) oder alternativ mittels MIM-Verfahren (Metal Injection Moulding) hergestellt werden. Bei diesem Verfahren wird ein Gemisch aus Metallpulver und organischem Binder in eine Form gespritzt, ausgeformt und abschließend gesintert. Bei diesem Verfahren wird der Flansch als Ganzes in einem Schritt gefertigt.

Was die Herstellung der Durchführung insgesamt anbelangt, so wird diese derzeit typischerweise aus relativ vielen einzelnen Komponenten (Isolationskeramik, Durchführungs-Flansch, Anschlusselement, Lot...) in mehreren Prozessen gefügt, wobei einige der Komponenten vorgelagerte thermische und Formgebungs-Prozesse durchlaufen, so dass die Herstellung einer Durchführung eine große Vielzahl an - teilweise bei hohen Temperaturen auszuführenden - Prozessschritten umfasst. Diese Verfahren sind arbeits- und energieintensiv und zudem relativ zeitaufwändig und daher insgesamt teuer.

Der Erfindung liegt die Aufgabe zu Grunde, eine verbesserte Durchführung der eingangs spezifizierten Art anzugeben, die insbesondere in einem vereinfachten Herstellungsprozess mit erhöhter Energieeffizienz und somit kostengünstiger hergestellt werden kann und mehr Freiheitsgrade hinsichtlich der Konstruktion bietet. Des Weiteren soll ein entsprechendes Herstellungsverfahren angegeben und ein relativ einfach und kostengünstig herzustellendes implantierbares medizinelektronisches Gerät bereitgestellt werden.

Diese Aufgabe wird in ihren Vorrichtungsaspekten durch eine Durchführung mit den Merkmalen des Anspruchs 1 bzw. durch ein medizinelektronisches Gerät mit den Merkmalen des Anspruchs 13 gelöst. In ihren Verfahrensaspekten wird die Aufgabe durch Verfahren mit den Merkmalen des Anspruchs 6 gelöst. Zweckmäßige Fortbildungen sind Gegenstand der jeweiligen abhängigen Ansprüche.

Die Erfindung schließt den Gedanken ein, den herkömmlichen Aufbau einer Durchführung dahingehend zu ändern, dass man grundsätzlich mit einer kleineren Zahl an Einzelkomponenten und einer kleineren Anzahl an einzelnen Prozessschritten auskommt. Insbesondere soll gemäß einem weiteren Gedanken der Erfindung, durch teilweise konstruktive Vereinigung von Einzelkomponenten im Rahmen vorgelagerter Prozessschritte die Anzahl späterer Fügeschritte verringert werden können. Dies betrifft, gemäß einem noch weiteren Gedanken der Erfindung, den Aufbau von Isolationskörper und Anschlusselementen bzw. Teilen hiervon als integrales Vorprodukt. Durch spätere thermische Behandlung dieses integralen Vorprodukts sollen und können traditionell getrennte thermische Prozessschritte für Isolationskeramik und Anschlusselemente zusammengefasst werden.

Letztlich ist erfindungsgemäß vorgesehen, dass der Isolierkörper aus Mehrlagenkeramik mit eingesinterten vorkonfigurierten Leiterelementen ausgebildet und mit einer gerätespezifischen Kontaktierung ausgewählter Leiterelemente versehen ist, die zusammen mit den kontaktierten vorkonfigurierten Leiterelementen die Anschlusselemente bildet. Spezieller handelt es sich beim Isolierkörper um eine Hochtemperatur-Mehrlagenkeramik (HTCC) deren Technologie mittlerweile bei der Herstellung von Durchführungen für medizinelektronische Geräte etabliert und an die Erfindung unproblematisch anpassbar ist.

In einer Ausführung der Erfindung ist der Isolierkörper als Bruchstück eines vorgefertigten Isolierkörper-Arrays ausgebildet. Diese Ausführung ermöglicht in noch weitergehender Weise als das erfinderische Grundkonzept eine Zusammenfassung von Prozessschritten für Einzelkomponenten und eine hiermit verbundene Einsparung an Energie und Prozessdauer. Zweckmäßigerweise ist das vorgefertigte Isolierkörper-Array mit Sollbruchstellen konfiguriert, die ein leichtes und insbesondere werkzeugloses Separieren der einzelnen Isolierkörper ermöglichen; diese Ausführung ist jedoch hierauf nicht beschränkt.

In einer weiteren Ausführung ist vorgesehen, dass der Durchführungs-Flansch ein Metallpulver-Spritzgussteil ist, welches um den Isolierkörper herum gespritzt und auf diesen auf geschrumpft ist. Auch hiermit wird das Konzept der Erfindung konsequent weiter ausdetailliert, indem die separate Vorfertigung eines Durchführungs-Flansches und ein gesonderter Schritt des Fügens von Isolierkörper und Flansch entfallen und der entsprechende Handhabungs- und Montageaufwand entfallen können.

In einer weiteren Ausführung umfasst die gerätespezifische Kontaktierung Dickschicht-Kontaktelemente in Art bekannter Strukturen der Dickschicht- bzw. Hybrid-Elektronik. In dieser Ausführung kann vorteilhaft auf bekannte und bewährte Konstruktions- und Verfahrenselemente der Dickschicht-Elektronik zurückgegriffen werden, ohne dass konstruktive oder technologische Neuentwicklungen und entsprechende Erprobungen und Funktionsnachweise erforderlich wären.

In einer weiteren Ausgestaltung des Konzepts der Erfindung umfasst die gerätespezifische Kontaktierung selektiv auf die vorkonfigurierten Leiterelemente aufgebrachte separate Kontaktelemente, insbesondere Anschlussstifte. Zwar kommen hier wiederum separate Komponenten ins Spiel, diese lassen sich aber in vorteilhaft integrierbaren Abläufen an den mit der gerätespezifischen Kontaktierung versehenen Isolierkörper anbinden, ohne dass die Komplexität des Herstellungsverfahrens wesentlich erhöht würde. Die Ausgestaltung ermöglicht zudem eine Realisierung des erfinderischen Konzepts in Verbindung mit weitgehend herkömmlichen Anschlussgeometrien des medizinelektronischen Gerätes, ohne wesentliche konstruktive Änderung derselben.

In einer weiteren Ausführung realisieren die vorkonfigurierten Leiterelemente oder ein Teil hiervon eine Filterfunktion der Durchführung, etwa in Art eines üblicherweise Anschlussstiften zugeordneten Filterkondensators. In einer Ausgestaltung wird diese Funktion unter Einbeziehung von Elementen der selektiv aufgebrachten gerätespezifischen Kontaktierung realisiert.

Unter Verfahrensaspekten schließt die Erfindung den Gedanken ein, dass der Isolierkörper mit den vorkonfigurierten Leiterelementen in einem HTCC-Verfahren gebildet und anschließend mit der gerätespezifischen Kontaktierung versehen und mit dem Durchführungs-Flansch umgeben wird. Spezieller ist hierbei vorgesehen, dass der Isolierkörper im getrockneten oder "grünen" Zustand in eine an die Form des Durchführungs-Flansches angepasste Spritzgussform eingebracht und durch Einspritzen von Metall in die Spritzgussform mit dem Flanschabschnitt umspritzt wird und anschließend der so vorgefertigte Verbund aus Isolierkörper und Durchführungs-Flansch gemeinsam entbindert und gesintert wird. Hierzu wird speziell als Material für den Durchführungs-Flansch ein solches, insbesondere Titan, gewählt, welches gegenüber dem Material des Isolierkörpers, insbesondere Aluminiumoxid, einen höheren thermischen Ausdehnungskoeffizienten hat, wodurch ein Aufschrumpfen des Durchführungs-Flansches auf den Isolierkörper beim Sintern bewirkt wird. Wird Titan als Durchführungs-Flansch-Material gewählt, so wird bevorzugt unter Sauerstoffausschluss (im Vakuum oder in Wasserstoff) gesintert, um einer Oxidation durch die hohe Sauerstoffaffinität des Titans entgegenzuwirken. Um gleichzeitig ein unerwünschtes diskontinuierliches Kornwachstum unter Bildung von sehr großen Kristallen im Aluminiumoxid zu unterdrücken wird speziell eine Dotierung mit MgO vorgesehen. Die Ausführung ist jedoch nicht auf die genannten Materialien beschränkt, sondern ebenso praktikabel mit anderen Materialkombinationen, die der genannten Bedingung für die thermischen Ausdehnungskoeffizienten genügen.

Die Erzeugung der gerätespezifischen Kontaktierung ist in sinnvoller Weise in verschiedenen Varianten möglich: Zum einen kann sie nach dem Umgeben des Isolierkörpers mit dem Durchführungs-Flansch ausgeführt werden. Zum anderen kann ein Dickschicht-Kontaktierungsschritt (etwa mit einer Maskenstruktur oder durch selektiven Schichtauftrag) ausgeführt werden. Schließlich ist, in einer weiteren Ausgestaltung, zur gerätespezifischen Kontaktierung ausgewählter Leiterelemente ein Bestücken mit einzelnen Kontaktelementen, insbesondere durch Auflöten, vorgesehen. Alle genannten Schritte bzw. Technologien sind an sich aus dem Bereich der keramischen Dickschicht- bzw. Hybridschaltungen dem Fachmann bekannt, so dass hier von einer genaueren Beschreibung abgesehen werden kann.

Vorteile und Zweckmäßigkeiten der Erfindung ergeben sich im Übrigen aus der Beschreibung von Ausführungsbeispielen anhand der Figuren. Von diesen zeigen:
- Fig. 1: eine schematische, teilweise geschnittene Darstellung eines implantierbaren medizinelektronischen Geräts,
- Fig. 2: eine schematische Darstellung, teilweise als Längsschnittdarstellungen, des Herstellungs-Ablaufes und Aufbaus einer Ausführungsform der erfindungsgemäßen Durchführung.

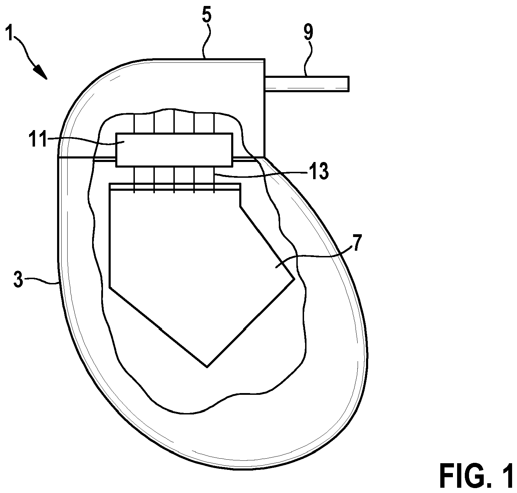

Fig. 1 zeigt einen Herzschrittmacher 1 mit einem Schrittmachergehäuse 3 und einem Kopfteil (Header) 5, in dessen Innerem neben anderen elektronischen Komponenten eine Leiterplatte (PCB) 7 angeordnet und mit dessen im Header angeordneten (nicht gezeigten) Leitungsanschluss eine Elektrodenleitung 9 verbunden ist. Eine zwischen Gerätegehäuse 3 und Header 5 vorgesehene Durchführung 11 umfasst eine Mehrzahl von Anschlusselementen 13. Die Anschlussstifte sind an einem Ende durch eine entsprechende Bohrung in der Leiterplatte gesteckt und mit dieser weich verlötet.

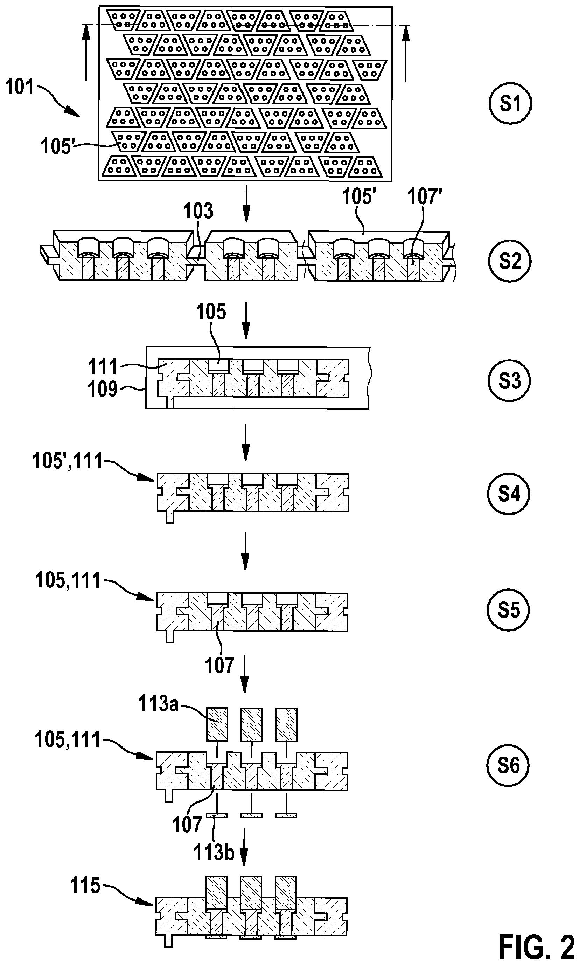

Fig. 2 zeigt schematisch, in Art eines mit Querschnittsdarstellungen der Durchführung verknüpften Ablaufschemas, schematisch einerseits den grundsätzlichen Aufbau eines Ausführungsbeispiels der erfindungsgemäßen Durchführung und andererseits wesentliche Schritte der Herstellung dieser Durchführung. Es wird darauf hingewiesen, dass die Darstellung rein schematisch ist und weder Details noch Größenrelationen einzelner Teile korrekt wiedergeben soll. Es wird auch darauf hingewiesen, dass die Schrittfolge des Herstellungsverfahrens hier keineswegs vollständig abgebildet ist.

Zunächst wird in einem Schritt S1 mittels der als solche bekannten HTCC-Technologie ein Isolierkörper-Array 101 aus einer Vielzahl regulär angeordneter, an Sollbruchstellen 103 miteinander verbundener Roh-Isolierkörper 105' mit eingebetteten Leiterelementvorstufen 107' erzeugt. Basis ist eine Aluminiumoxid-Keramik, und für die eingebetteten Leiterelemente kann ein aus der Hybridtechnologie an sich bekanntes Material für Leiterbahnen, etwa auf Silber- oder Kupferbasis, benutzt werden.

Im noch "grünen" Zustand werden aus dem Isolierkörper-Array 101 in einem Schritt S2 die Isolierkörper 105 durch Brechen an den Sollbruchstellen 103 vereinzelt. In einem nachfolgenden Schritt S3 wird der vereinzelte Roh-Isolierkörper 105' in eine Metallspritzgussform 109, in der ein Form-Hohlraum in der Gestalt eines Durchführungs-Flansches vorgesehen ist, eingelegt und mittels der MIM-Technologie mit Titan umspritzt, das im Form-Hohlraum einen den Roh-Isolierkörper 105' umgebenden Durchführungs-Flansch 111 ausbildet. In einem weiteren Schritt S4 wird das vorgefertigte integrale Bauteil Isolierkörper/Flansch 105'/111 gemeinsam entbindert, und in einem weiteren Schritt S5 erfolgt ein gemeinsames Sintern bei Temperaturen T>1300°C bevorzugt in reduzierender Atmosphäre, wobei in Folge des etwas höheren Wärmeausdehnungskoeffizienten des Flansch-Materials Titan ein Aufschrumpfen auf den aus AL2O3 bestehenden Isolierkörper-Abschnitt erfolgt. Zugleich entsteht aus dem Roh-Isolierkörper 105' der fertige Isolierkörper 105, mit vollständig leitfähig ausgeprägten metallischen Leiterelementen 107.

In einem optionalen weiteren Schritt S6 wird auf den im Schritt S5 als solchen fertiggestellten integralen Isolierkörper/Flansch 105/111 eine gerätespezifische Kontaktierung auf gebracht. Dies ist in der Figur durch beidseitiges Aufbringen erster und zweiter Kontaktelemente 113a, 113b auf sämtliche Leiterelemente 107 symbolisiert, jedoch ist weder die beidseitige zusätzliche Kontaktierung noch eine Kontaktierung sämtlicher vorgefertigter, eingebetteter Leiterelemente zwingend. Die Anbringung kann beispielsweise durch Löten oder Bonden oder Beschichten, zum Beispiel mit einem Ag-Kolloid, erfolgen. Falls eine nachträgliche Verlötung mit Nb Anschlussstiften erforderlich wäre, könnte mittels eines aktiven Lotes (zum Beispiel Ti Cu Ni) der HTCC-Isolierkörper vorkonditioniert werden. Es ergibt sich insgesamt eine zur Herstellung der erforderlichen elektrischen Verbindungen eines speziellen medizinelektronischen Gerätes geeignet konfigurierte Durchführung 115, die in einem Herstellungsverfahren mit vereinfachtem Ablauf, geringerem Handling, Auf wand und geringerem Energieverbrauch hergestellt wurde.

Die Ausführung der Erfindung ist auch in einer Vielzahl von Abwandlungen der hier gezeigten Beispiele und weiter oben hervorgehobenen Aspekte der Erfindung möglich.

## Patentansprüche

1. Durchführung (11; 115) eines, insbesondere implantierbaren, medizinelektronischen Gerätes (1) mit einem Gehäuse (3) und mindestens einem in dem Gehäuse aufgenommenen elektrischen oder elektronischen Bauelement (7), wobei die Durchführung einen Durchführungs-Flansch (111) zum Verschluss einer Öffnung des Gehäuses und zur Aufnahme einer Mehrzahl von Anschlusselementen (13; 107, 113a, 113b), die zum gehäuse-externen Anschluss des oder mindestens eines Bauelementes dienen, in einem die Anschlusselemente umgebenden Isolierkörper (105) aufweist, wobei der Isolierkörper aus Mehrlagenkeramik insbesondere aus HTCC, mit eingesinterten vorkonfigurierten Leiterelementen (107) ausgebildet und mit einer gerätespezifischen Kontaktierung (113a, 113b) ausgewählter Leiterelemente versehen ist, die zusammen mit den kontaktierten vorkonfigurierten Leiterelementen die Anschlusselemente bildet.

2. Durchführung nach Anspruch 1, wobei der Isolierkörper (105) als Bruchstück eines Isolierkörper-Arrays (101) ausgebildet ist.

3. Durchführung nach Anspruch 1 oder 2, wobei der Durchführungs-Flansch (111) ein Metallpulver-Spritzgussteil ist, welches um den Isolierkörper (105) herum gespritzt und auf diesen aufgeschrumpft ist.

4. Durchführung nach einem der vorangehenden Ansprüche, wobei die vorgefertigten Leiterelemente (107) und/oder die gerätespezifische Kontaktierung (113a, 113b) Dickschicht-Kontaktelemente umfassen.

5. Durchführung nach einem der vorangehenden Ansprüche, wobei die gerätespezifische Kontaktierung (113a, 113b) selektiv auf die vorkonfigurierten Leiterelemente (107) aufgebrachte separate Kontaktelemente, insbesondere Anschlussstifte (113a), umfasst.

6. Durchführung nach einem der vorangehenden Ansprüche, wobei die vorkonfigurierten Leiterelemente (107), optional in Verbindung mit der gerätespezifischen Kontaktierung (113a, 113b), eine Filtereinrichtung der Durchführung realisieren.

7. Verfahren zur Herstellung einer Durchführung (11; 115) nach einem der vorangehenden Ansprüche, wobei der Isolierkörper (105) mit den vorkonfigurierten Leiterelementen (107) in einem HTCC-Verfahren gebildet und anschließend mit der gerätespezifischen Kontaktierung (113a, 113b) versehen und mit dem Durchführungs-Flansch (111) umgeben wird.

8. Verfahren nach Anspruch 7, wobei der Isolierkörper (105) als Abschnitt eines Isolierkörper-Arrays (101) gebildet und anschließend vereinzelt wird.

9. Verfahren nach Anspruch 7 oder 8, wobei der Isolierkörper (105') im getrockneten oder "grünen" Zustand in eine an die Form des Durchführungs-Flansches angepasste Spritzgussform (109) eingebracht und durch Einspritzen von Metall in die Spritzgussform mit dem Durchführungs-Flansch umspritzt wird und anschließend der so vorgefertigte Verbund (105'/111) aus Roh-Isolierkörper und Durchführungs-Flansch gemeinsam entbindert und gesintert wird.

10. Verfahren nach Anspruch 9, wobei als Material für den Durchführungs-Flansch (111) ein solches, insbesondere Titan, gewählt wird, welches gegenüber dem Material des Isolierkörpers, insbesondere Aluminiumoxid, einen höheren thermischen Ausdehnungskoeffizienten hat, wodurch ein Aufschrumpfen des Durchführungs-Flansches auf den Isolierkörper beim Sintern bewirkt wird.

11. Verfahren nach Anspruch 10, wobei der Durchführungs-Flansch (111) aus Titan und der Isolierkörper (105) aus Aluminiumoxid gebildet wird, wobei der Roh-Isolierkörper und Durchführungs-Flansch gemeinsam unter Sauerstoffausschluss gesintert werden und insbesondere der Roh-Isolierkörper mit Magnesiumoxid dotiert wird.

12. Verfahren nach einem der Ansprüche 7 bis 11, wobei die gerätespezifische Kontaktierung (113a, 113b) ausgewählter vorkonfigurierter Leiterelemente (107) nach dem Umgeben des Isolierkörpers (105) mit dem Durchführungs-Flansch (111) ausgeführt wird.

13. Verfahren nach einem der Ansprüche 7 bis 12, wobei zur gerätespezifischen Kontaktierung ausgewählter Leiterelemente (107) ein Dickschicht-Kontaktierungsschritt ausgeführt wird.

14. Verfahren nach einem der Ansprüche 7 bis 12, wobei zur gerätespezifischen Kontaktierung ausgewählter Leiterelemente (107) ein Bestücken mit einzelnen Kontaktelementen (113a, 113b), insbesondere durch Auflöten, ausgeführt wird.

15. Medizinelektronisches Gerät (1) mit einer Durchführung (11; 115) nach einem der Ansprüche 1 bis 6, insbesondere ausgebildet als Herzschrittmacher, Kardioverter oder Cochlear-Implantat.
